# EUROPEAN PATENT SPECIFICATION

(11) **EP 2 152 220 B1**
(45) Date of publication and mention of the grant of the patent: **28.09.2011**
(21) Application number: 08737248.8
(22) Date of filing: 02.05.2008
(51) Int. Cl.: A61H 23/02

(54) **VIBRATION PAD COVER AND VIBRATION TREATMENT SYSTEM**
ABDECKUNG FÜR EINE VIBRATIONSAUFLAGE UND VIBRATIONSBEHANDLUNGSSYSTEM
ENVELOPPE DE COUSSIN VIBRATOIRE ET SYSTÈME DE TRAITEMENT À VIBRATION

(30) Priority: 03.05.2007 GB 0708575
(43) Date of publication of application: 17.02.2010
(73) Proprietor: Vibrant Medical Limited, Sheffield S1 4DP (GB)
(72) Inventor: ELLIN, Philip James, South Yorkshire S65 4RB (GB)
(74) Representative: Lunt, Mark George Francis
(86) International application number: PCT/GB2008/050324
(87) International publication number: WO 2008/135788

(56) References cited:
- WO-A-02/065973
- US-A- 2 917 043

## Description

This invention relates to a cover for a vibration pad and to a vibration treatment system employing such a cover.

### BACKGROUND

Our co-pending application number WO-A-02065973 discloses a method of treatment of lymphodema and leg ulcers and a prophylactic treatment for deep vein thrombosis (DVT) employing mechanical vibrations, particularly cycloidal vibration, employing a vibration pad. Cycloidal vibration is a small amplitude, 0.1 and 0.5mm, low frequency, 15 to 75HZ, vibration that produces motion in three different directions, each of these directions will be at different points in its cycle. It is the out of "phase" relationship which gives rise to the term cycloid vibration. GB-A-2096899 and US3019785 disclose a vibration pad device comprising a motor mounted in a frame, the frame extending into a pad and the motor driving an eccentric weight that causes cycloid vibration of the pad. Cycloidal vibration can be administered by means of integration of the mechanism into static products such as a portable pad as disclosed in GB-A-2096899, but equally it can be incorporated in a mattress, of a therapy couch, for example.

US-A-2006247601 relates to the treatment of cellulitis. Cellulitis is a common skin infection. In 2002 to 2003, in the UK, there were nearly 60,000 recorded admissions into hospital. Each admission can take on average 10 days to treat (2), accounting for up to six hundred thousand-bed days per annum. Most commonly affecting the lower limbs, cellulitis is an acute infection of the skin and subcutaneous tissues, characterised by: local heat, redness, pain, erythematous tissue and swelling. It is commonly caused by the bacteria streptococci and is associated with, or can be a consequence of, lower limb swelling/oedema. This can be due to a mix of any of the following: leg oedema, venous hypertension, lymphodema, chronic ulceration and immobility.

US-A-2006247601 provides a method of treatment of cellulitis comprising the steps of administering one or more antibiotics and applying a vibration pad to the region of the skin affected by cellulitis and submitting the pad to cycloid vibration for a period of at least 30 minutes at least once per day until the infection diminishes. The vibrations are believed to assist transport of the antibiotics to the site of infection so that they have their effect more rapidly and completely.

Leg ulcers, lymphodema and cellulitis all result, to a greater or lesser extent, in exudation from the skin of potentially infectious material. WO-A-02065973 proposes the use of a cover for the vibration pad, so that exudates can be isolated from the pad and any weeping or bleeding of wounds can be absorbed by the cover and not infect the surface of the pad. However, there is a strong tendency in all environments to reuse apparatus and in today's environmentally-conscious society the temptation not to waste disposable medical supplies can sometimes lead to risks being taken. In fact, the danger of cross-infection is far more costly, even just in environmental terms, than a strict adherence to a single-use policy regarding medical products, and this includes covers of vibration pads.

It is an object of the present invention to provide a cover including means to substantially guarantee single use thereof, or at least to make it difficult to reuse.

### BRIEF SUMMARY OF THE DISCLOSURE

In accordance with the present invention as claimed there is provided a cover for a vibratory pad comprising a pocket faced with dressing fabric material and sized to accommodate a vibration pad, a closure to retain the cover on the pad, in use, and a strap integral with the cover suitable for connecting the cover and a pad retained therein to the limb of a patient, said strap having a connector that is capable of being secured so as to pressure the pad against the limb and so that, once made, the connection cannot be unmade without disabling the connector against making subsequent connections. Thus the cover can only be used once.

From an infection control perspective, and because the cover and straps are in direct contact with infected skin that is often leaking exudate/fluid, particularly during vibration treatments, it is undesirable that the recovering skin/limb should be in contact with the same cover and straps that may have been contaminated with bacteria-infected skin cells or fluid from earlier stages of the treatment. Replacement of the cover per treatment session may aid recovery and assist in preventing reoccurrence of infection.

Preferably said connector comprises a strip of adhesive and said material of the cover is of the type to which the adhesive adheres non-releasably. Here, non-releasably means that the adhesion between the strap and the material of the cover is such that the connection between the strap and cover cannot be unmade without losing the capacity of the adhesive to adhere the strap again to the material of the cover.

Thus the adhesive may be so strong that the adhesive bond cannot be broken at all, and that, in order to disconnect the pad from a patient's leg, the strap must be broken or cut.

Alternatively, the material of the cover may be layered, whereby when the strap is disconnected from the cover, a surface layer of the cover is detached from the cover remaining adhered to the strap, whereby the strap no longer has capacity to secure the pad to a patient's limb. In this sense, "layered" does not necessarily mean that discrete layers exist in the material of the cover but only that what elements of the cover that become adhered to the strap have greater cohesion to the adhesive of the strap than to the remainder of the cover and detach therefrom on peeling of the strap.

Alternatively, the adhesive of the strap may have greater cohesion to the material of the cover than to the remaining material of the strap. In this event, on peeling of the strap, the adhesive may be left attached to the cover, but like in the previous alternative, in accordance with the present invention, material of the strap must detach from the strap to remain connected to the adhesive so as to destroy the capacity of the adhesive to effect another connection to the strap.

Preferably, the adhesive is protected by a release layer prior to use.

Preferably, said cover comprises two sheets of material connected together along three edges to form a pocket, a fourth edge of one sheet of said two sheets having an extended flap provided with a closure whereby a pad inserted in the open mouth of the pocket formed between said fourth edges of the sheets may be retained therein. Preferably, said closure is of the type that, once closed cannot be opened without disabling the closure against making subsequent closure. Preferably said closure comprises a strip of adhesive and said material of the cover is of the type to which the adhesive adheres non-releasably.

Preferably, one of said sheets includes at least one extension from one of said three sides and forming said strap. Preferably, said extension is from a first side of the sheet adjoining said fourth side. Preferably, two of said extensions are formed from the same side providing two straps. Preferably, the third side is also provided with a strap or straps corresponding with that or those of the first side. Preferably, said closure flap extends from one of said sheets being a front sheet, and the strap or straps extend from the other of said sheets being a rear sheet, the front sheet being the sheet intended, in use, to be against a patient's leg.

Preferably, said material of the cover comprises a waterproof layer and at least one absorbent layer. Said waterproof layer may be a sheet of plastics material, preferably a thermoplastic material, such as polyethylene. Said absorbent layer may comprise a fibrous flock adhered to said waterproof layer. Said fibrous flock may be paper. Said connection between said sheets of material may be by welding said thermoplastic components of the sheets to each other. Preferably, said waterproof layer is sandwiched between two of said absorbent layers.

Said cover may comprise a single sheet and said closure may comprise a draw string whereby the sheet is formable into said pouch to accommodate the pad. The drawstring may be a closed loop of elasticated material, in which event the pouch is snapped over the vibration pad to fit it.

Indeed, said cover may comprise a single sheet and said closure may comprise said connector, which is in the form of a strap extending from a side of said sheet and adapted to wrap around said pad and said limb, and secure to said sheet, and whereby the sheet is formable into said pouch to accommodate the pad.

Alternatively, the connector could be in the form of at least two pairs of straps extending from either side of said sheet and adapted to wrap around said pad and said limb, and whereby the sheet is formable into said pouch to accommodate the pad. In this event, the straps cross over one another, and so a single pair would not provide an stability for the connection.

In a different aspect, the present invention provides a vibration treatment system comprising a vibration device and a cover therefor, wherein the vibration device comprises a motor driving a vibration element and a pad connected to the motor whereby vibrations caused by rotation of the motor are transmitted to and by the pad, a controller controlling operation of the motor and including a first interface element, and wherein the cover comprises sheet material to protect the pad against contamination when the system is in use, the cover including a second interface element and a disabler, and wherein said first and second interface elements are interengageable on application of the cover to the pad whereby said controller detects the presence of the cover and enables operation of the motor for a period of time until the controller and disabler disable further operation of the motor until a different cover is applied to the pad.

Said first and second interfaces comprise a radio frequency transmission system. In this event, said disabler may comprise electric circuitry associated with the cover.

In one embodiment, said circuitry includes a unique identification code device that is read by the controller on interengagement of said first and second interfaces and entered into a memory forming part of the controller whereby, if the code is already in the memory the controller disables the motor from operating. If the code is not already in the memory, it is stored in the memory and the motor is enabled to operate for a period of time.

Said period of time may be a period of time suitable for a single period of therapy using the vibration device. Once the period has elapsed, the cover incorporating the second interface device and disabler are no longer able to operate with the vibration device and a new cover is required instead.

Said second interface and disabler may comprise a known passive radio frequency identification (RFID) tag that has no internal power supply. The vibration device may incorporate the known arrangements (reader) to activate and interrogate the RFID, but only over a very short distance commensurate with the cover being disposed on the vibration device. In such RFIDs, an antenna collects radio frequencies and the minute electrical current induced in the antenna by the incoming radio frequency signal provides just enough power for eg a CMOS integrated circuit in the tag to power up and transmit a response. Most existing passive tags signal by backscattering the carrier wave received from the reader. This means that the antenna is designed both to collect power from the incoming signal and also to transmit the outbound backscatter signal. The response of a passive RFID tag is not necessarily just an ID number; the tag chip can contain non-volatile, possibly writable EEPROM for storing data.

For example, one arrangement provides that a cover incorporating a tag is disposed on the vibration device. When the patient is ready, a button or other start signal is activated on the vibration device. The vibration device then transmits a signal to the RFID tag in the cover. The tag responds with a code that enables the vibration device to commence operation. At the same time, a clock in the in RFID begins to count down and, after a period of time, the code response is stopped by the RFID tag and the motor in the vibration device stops.

The advantage of this arrangement is that the vibration device does not require a large memory to store codes associated with previously used covers. Indeed, a used cover could not be reused on a different vibration device. Furthermore, covers could be tailored for specific uses, having different treatment times, and possibly having different physical characteristics, depending on the use. For example, where the cover is to be used for ulcer treatment or cellulitis, where wound weeping is a significant risk, the treatment time may be set quite short, probably limited to a single treatment period, to minimise the risk of cross- or re- infection; and the surface of the cover may be absorbent to retain any seepage. On the other hand, for deep vein thrombosis prophylactic treatment, the treatment time may be longer, or permit multiple treatments, and the cover not so absorbent. Moreover, the code transmitted by the cover may not only enable the vibration device but may inform the vibration device of the treatment regime to be employed, and the vibration device may operate at different levels of vibration, or in different modes, depending on the treatment being effected.

Passive RFID tags have practical read distances ranging from about 10 cm (4 in.) (ISO 14443) up to a few meters (Electronic Product Code (EPC) and ISO 18000-6), depending on the chosen radio frequency and antenna design/size. In the present invention, only a short range is required. Due to their simplicity in design they are also suitable for manufacture with a printing process for the antennas. The lack of an onboard power supply means that the device can be quite small: commercially available products exist that can be embedded in a sticker, or under the skin in the case of low frequency RFID tags.

Passive RFID tags are currently available with privacy enhancing technologies built-in, including built-in firewall access controls, and communication encryption. The ongoing problem with all RFIDs is that they need an external antenna which is 80 times bigger than the chip in the best version thus far developed. Nevertheless, this is not an issue with a cover for a vibration device that has plenty of surface area available. Further, the present costs of manufacturing tags has inhibited broader adoption. As silicon prices are reduced and new more economic methods for manufacturing inlays and tags are perfected in the industry this option is more likely to be relevant.

A further alternative arrangement of first and second interface is the use of induction coils. Indeed, this technology is currently employed with contactless smart cards. An integrated circuit chip communicates with the card reader through induction technology (at data rates of 106 to 848 kbit/s). These cards require only close proximity to an antenna to complete transaction. The standard for contactless smart card communications is ISO/IEC 14443, dated 2001. It defines two types of contactless cards ("A" and "B"), allows for communications at distances up to 10 cm.

Preferably, said first and second interfaces comprise a simple plug and socket, wherein the disabler comprises electric circuitry associated with the cover. The arrangements described above with reference to RFID tags are equally applicable here, the only different being that, instead of a transmitter and antenna, the interfaces are a plug and socket, but the functionality of the disabler can be exactly the same as with an RFID as described above. This arrangement is simpler in many respects and easier for a user to understand, and may be preferred.

Alternatively, however, the disabler in a simple plug and socket arrangement may comprise a fuse resistor, the circuit in the vibration device detecting the resistance of the fuse resistor when connection between the interfaces is made and, provided that the detected resistance is within a predetermined range of resistances, the motor is enabled to operate. However, a timer in the vibration device is arranged to send a current pulse to the fuse resistor to "blow" the fuse so that it goes into open circuit. Thereafter, no resistance is detected and the cover can no longer be employed. Again, the level of resistance may be employed to distinguish between covers for different applications and to tailor the time of permitted operation of the motor with that cover before the vibration device sends the signal blowing the fuse.

Any suitable disablement arrangement is contemplated. Indeed, in its broadest aspect, what the present invention provides is a vibration treatment system comprising a vibration device and a cover therefor, wherein the vibration device comprises a motor driving a vibration element and a pad connected to the motor whereby vibrations caused by rotation of the motor are transmitted to and by the pad, and the cover comprises sheet material to protect the pad against contamination when the system is in use and the cover is applied to the pad, wherein the system further comprises disablement means to disable use of the vibration device with a particular cover once that cover has been employed in a treatment regime.

In its simplest form, said disablement means comprises a strap integral with the cover suitable for connecting the cover when applied to a pad to the limb of a patient, said strap having a connector that is capable of being secured so as to pressure the pad against the limb and so that, once made, the connection cannot be unmade without disabling the connector against making subsequent connections. Thus the cover can only be used once.

However, in more sophisticated forms, said disablement means comprises a controller controlling operation of the motor and including a first interface element, and wherein the cover includes a second interface element and a disabler, and wherein said first and second interface elements are interengageable on application of the cover to the pad whereby said controller detects the presence of the cover and enables operation of the motor for a period of time until the controller and disabler disable further operation of the motor until a different cover is applied to the pad.

By virtue of the present invention, the hygiene arrangements around the use of vibration therapy for treatment of a range of conditions, a number of which carry a risk of infection and contamination, can be more assuredly provided.

### BRIEF DESCRIPTION OF THE DRAWINGS

Embodiments of the invention are further described hereinafter with reference to the accompanying drawings, in which:
Figure 1 is a perspective view of a vibratory massage device of the type employed in the present invention, (having attached thereto a transducer pack analysing the vibrations of the pad in three orthogonal directions x, y and z);
Figure 2 is a side view of the device of Figure 1 strapped to a patient's leg with the drive unit at the heel of the patient;
Figure 3 is a similar view to Figure 2, but with the drive unit under the knee of the patient;
Figure 4 is an assembly drawing of a drive unit and frame of the device of Figure 1, a cover of the drive unit, and the padding of the frame being removed;
Figure 5 is a perspective view of the device of Figure 1, with the casing of the drive unit open;
Figure 6 is a perspective view of a cover in accordance with the present invention;
Figures 7a and b are plan views of two further embodiments of the present invention, ready to wrap a vibration pad, the embodiment of Figure 7a having two overlapping strap pairs, while Figure 7b has a single strap;
Figure 8 is a perspective inverted view of the arrangement of Figure 7, the pad and cover being shown ready to receive the limb of a patient;
Figure 9 being a perspective inverted view of a further embodiment of a cover in accordance with the present invention, on a vibration pad; and
Figure 10 is a schematic illustration of a number of possible alternative arrangements of disablement arrangements of the second aspect of the present invention.

### DETAILED DESCRIPTION

In the drawings, a vibratory massage device 10 of the type employed with the present invention comprises a drive unit 12. The drive unit comprises a casing 14 housing an electric low voltage DC motor 16 mounted in the casing through flexible mountings 18,20. The motor drives an eccentric weight 22 mounted on a fan 23 on each end of an armature 24. On rotation of the armature 24 motor 16 imparts a vibration in the casing 14 in a radial plane (x, y) with respect to the armature 24. Because the mountings 18,20 are soft, a component of the vibration occurs in a direction orthogonal (z) to the radial plane. Consequently, the vibration of the casing in response to the vibration of the motor is three-dimensional.

To the casing is fixed, by screws (not shown) retained in apertures 25 of the casing, a frame 27. On the frame is disposed fabric cushioning to form a pad 110. The motor is adapted to rotate at about 2400 rpm providing a frequency of vibration of about 40 Hz.

Depending on various factors (primarily connected with the degree of restraint placed upon the device by its location on the limb of an animal) the amplitude of vibration in each direction may be different and between about 0.1 mm and 0.5 mm. However, a speed control arrangement (not shown) is provided to control the power supplied to the motor.

Because the frame 27 is rigidly fixed to the casing 14 of the drive unit 12, vibrations of the drive unit 12 are therefore transmitted to the pad 110. The pad is about 400 mm long and about 250 mm wide at the motor end and about 200 mm wide at its other end.

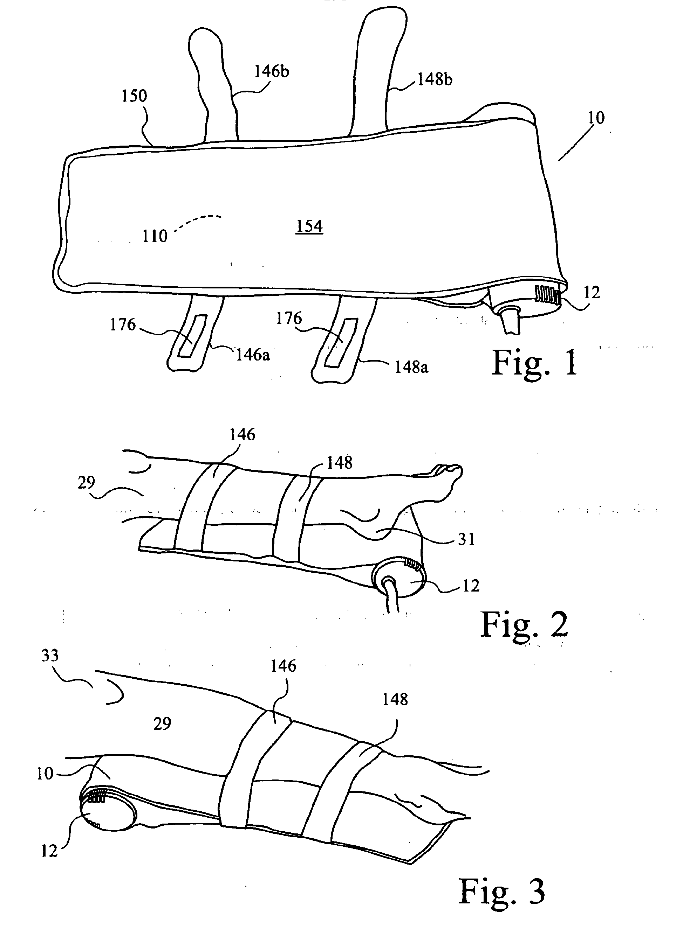

In use, a patient, suffering from a leg ulcer or cellulitis or some similar and potentially infectious condition, lays the affected leg 29 longitudinally along the pad. Whether the motor is at the heel end 31 of the leg, as shown in Figure 2, or is under the knee 33, as shown in Figure 3, is a matter of patient choice. However, if an ulcer is on the patient's ankle or lower leg, the former arrangement may be preferable, whereas if it is on the calf or higher, the latter arrangement may provide more direct delivery of vibrations to the site and environment of the ulcer.

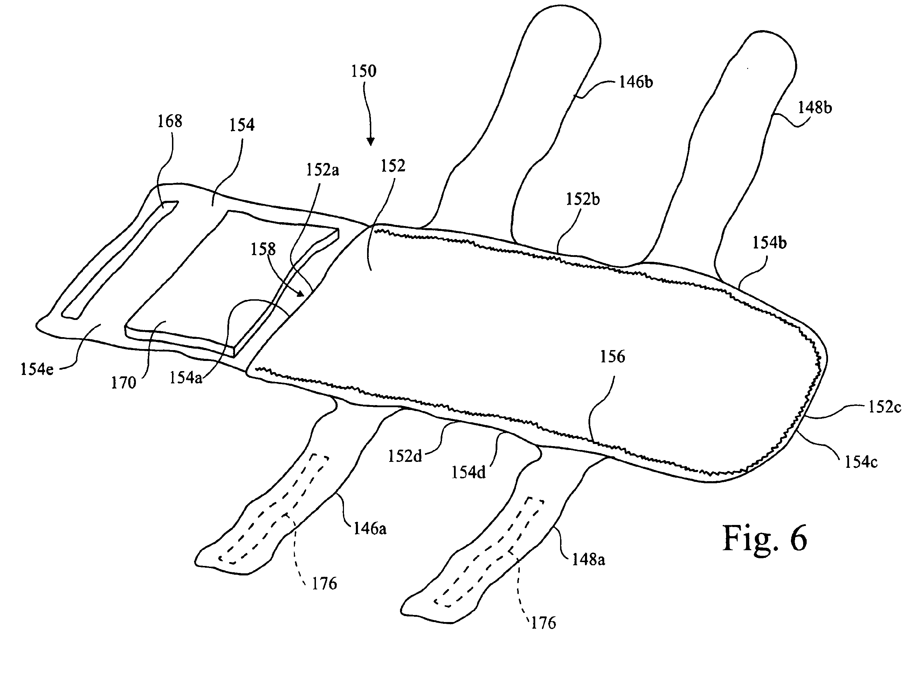

Turning to Figure 6 a cover 150 is illustrated comprising a pocket or pouch formed from two sheets 152,154 each of an impervious, but soft-feel fabric, material. Such a material is a paper flock covered polyethylene or polypropylene sheet as frequently currently used surgical environments. For example, such material is presently sold by Kimberly-Clark® as surgical drapes and gowns manufactured from polypropylene fabric with the benefits of low linting, ignition resistance and exceptional barrier properties for protection from airborne and blood borne bacteria. However, the precise form of the sheet is within the ambit of the person skilled in the art and does not form part of the present invention. Nevertheless, in the context of the present invention, "impervious" should be understood to mean that liquid weeping from a bandaged ulcer of a patient undergoing treatment with the device will not, on the whole, penetrate the material and contaminate the pad. However, a certain breathability of the material is certainly permitted. Thus, for the purposes of patient comfort, the cover may not be utterly impervious and therefore on occasions some contamination may happen if significant leakage occurs.

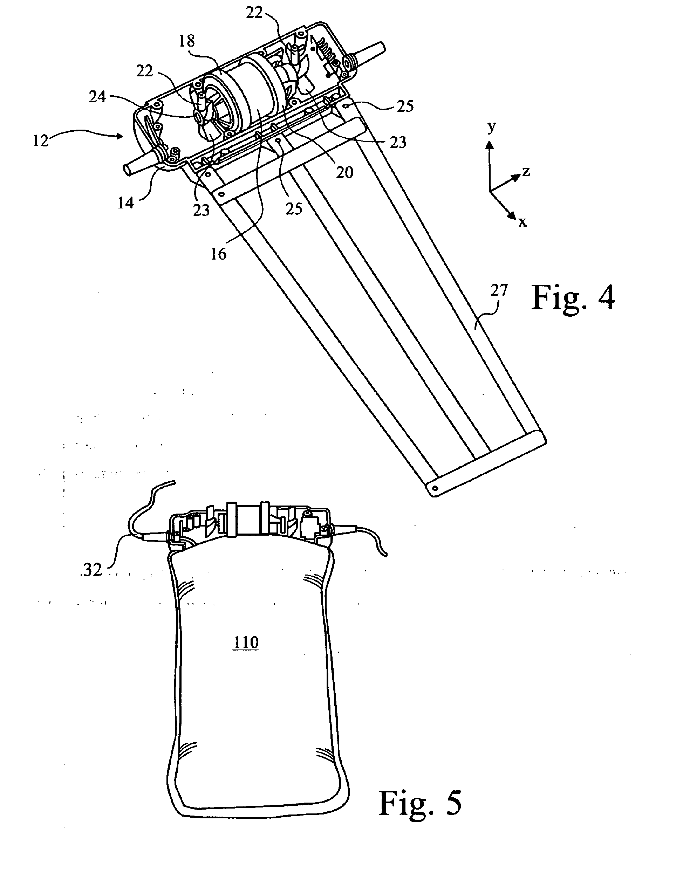

Sheet 154 forms a front of the cover 150, adapted to lie against the skin of the patients leg, whereas sheet 152 forms the back. Each sheet has, essentially, four sides 152a-d and 154a-d together forming the same shape and being connected together along joint line 156 to form an open pouch 158. First ends 152a,154a are not connected together and form the open mouth of the pouch 158. Second, third and fourth edges 152b-c are connected to corresponding edges 154b-c, preferably by heat welding. End 154a of front sheet 154 is provided with an extension 154e. The pouch 158 of the cover 150 is shaped to snugly receive the pad 110 of a massage device 10 of the type shown in Figures 1 to 5. The motor 12 is not received in the pouch 158, however. Instead, the extension flap 154e covers the motor when the flap is folded over. An adhesive strip 168 is provided on the flap 154e to close the pouch 158 and retain the pad 10 within the confines of the cover 150. The adhesive strip 168 is adapted to adhere against the face of sheet 152. A foam pad 170 wraps the motor 12 and isolates to a significant degree direct vibrations of the motor from the patient.

The cover 150 has two pairs of straps 146a,b and 148a,b integral with the front side 154. The straps 146a,b have adhesive strips 176 along their length. The strips are provided with protective release paper (not shown) to prevent inadvertent adhesion before they are ready. When a patient's leg is placed along the pad 110 (front face 154 of cover 150) the leg can be pressed against the device 10 by folding over the straps 146a,148a and engaging them with the other straps 146b,148b to form closed securing loops 146,148 (see Figures 2 and 3).

The straps 146b,148b could be omitted if desired, but then the straps 146a,148a would have to be longer. The pressure applying means that is in the form of the straps 146,148 is employed to press the leg into close contact with the pad 110 so that vibrations penetrate deeply and widely into the flesh of the patient's limb.

In its first aspect of the present invention, the adhesive strips 176, and also desirably the strip 168, are selected, in combination with the material of the sheets 152,154, so that, once the straps are secured in position, their subsequent detachment destroys their capacity to form a further bond. This is inconvenient in some respects, because it does not allow for any adjustment of the pressure applied by the straps once they have been connected. However, this disadvantage is outweighed by the need to be sure that a fresh cover is employed for each patient, and that the risk of cross-infection between patients is minimised. It is not doubted that medical staff are trained to observe and be aware of the need for good clinical hygiene, but the present invention is provided so that best practice is not only reliant on the good sense of the staff.

The arrangement may be one of three:
First - the adhesive bond and cohesive strength of the materials connected by the adhesive are so strong that the straps cannot be detached at all without breaking them, rendering the cover incapable again of connecting the pad to a patient's leg;
Second and third - the cohesive strength of the materials connected by the adhesive is less than the adhesive bond between those materials (or one of them) and the adhesive, so that, despite the strap being peelable, the adhesive remains connected to one or other of the surfaces, the other surface breaking down and detaching from the sheet or strap, as the case may be, rendering the adhesive strip without any tackiness for effecting a further adhesion.

A suitable combination of sheet material and adhesive is as follows:

### Sheet material:

The disposable cover consists of sleeve constructed from Microgard® 2000, a material produced by Microgard Limited of Hull, United Kingdom. The seams of the cover are reinforced and ultrasonically welded. Microgard® 2000 technical profile

| | | | |
|---|---|---|---|
| Abrasion | EN 530 (method 2) | >500 | Class 3 |
| Bursting | ISO 2960 | 167KpA | Class 2 |
| Tear | ISO 9073 | 39N (MD) | Class 1 |
| | | 25.7N(CD) | Class 1 |
| Fire retardency | EN1146:1997 | | Pass |
| Seam strength | EN13935-2:1999 | 106.1 N | Class 3 |
| | | | |
| Chemical | EN 368 | Repellency index | Penetration |
| Repellency | n-Heptane | 87.7% | 0.1% |
| | Isopropanol | 93.9% | 0.0% |
| | Sulphuric Acid 30% | 98.1% | 0.0% |
| | NaOH 10% | 98.5% | 0.0% |
| | | | |
| Type 5 | Reduced Spray Test - prEN13034:1997 | | Pass |
| Type 6 | Particle Penetration Suit test prENISO 13982 (1 &2) | | Pass |
| | | | |
| Surface Resistivity | EN 1149.1 | Conforms to all anti-static requirements | |
| Aloxite Penetration Test | | Particle Penetration through fabric only | |
| | | | |
| Royco Channel | 1.0 - 1.0 µm | <1% | |
| | 1.5 - 2.0 µm | <1% | |
| | 2.0 - 2.5 µm | <1% | |
| | 2.5 - 3.0 µm | <1% | |
| | 3.0 - 3.5 µm | <1% | |
| | >3.5 µm | no penetration | |

Fabric complies to CEN TC 162/WG3/TG3/N85 which requires particles in the size range 3.0 - 3.5 µm does not exceed 10% penetration.

### Adhesive:

A Double Bonded Tape to either one of the following specifications:
3M 9571, a high tack acrylic adhesive, hand-tearable double tape, with a tissue carrier on an easy release paper liner; or
3M 9087 a double coated tapes with 3M Adhesive 375, providing a high level of adhesive peel and shear performance. The adhesive system used provides good adhesion to both high and low surface energy substrates. The excellent initial tack ensures that a bond of good integrity is achieved soon after application.
These products are supplied by 3M United Kingdom plc, Bracknell, UK.

Such a combination results in the second/third arrangement described above.

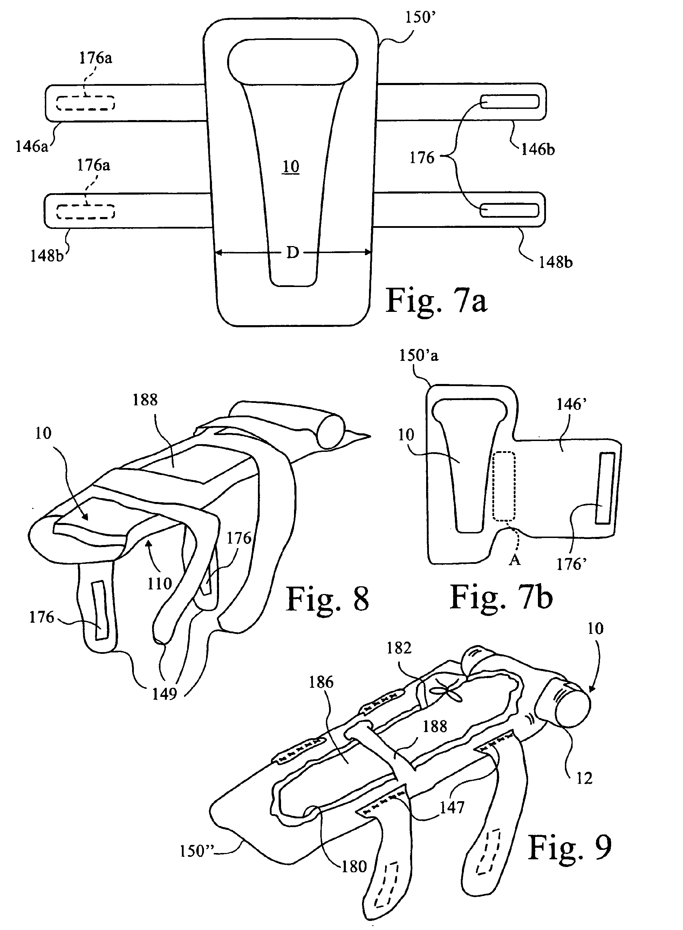

Figures 7 to 9 show further embodiments within the ambit of the present invention where the pouch or pocket is only formed when the pad is attached to the pad 10.

Thus, In Figure 7a, the cover 150' comprises a single sheet of, for example, Microgard® 2000, having at least two pairs of long straps 146a,b and 148a,b, one each of which (b) is provided with the adhesive strip 176. In this embodiment (although it could equally apply to the other embodiments described herein - as, indeed, those embodiments could apply here) further adhesive strips 176a are provided on the opposing straps (a). When joined, these form such a strong bond that they cannot be separated. To apply the cover 150', it is lain on a surface with its patient-facing surface (if it has one) face-down. The pad 10 is then placed face-down centrally on the cover, with its padded front face 110 (the face to be applied to the patient) against the cover 150'. The straps 146,148a,b are then folded over behind the pad 10 (as shown in Figure 8), and passed across each other to extend back towards front surface 110. The pad and loosely attached cover is then turned over and a patient's limb is laid on the covered front face 110. The strap ends 149 are then passed over the limb and, after adjusting for tightness and comfort, the adhesive strips 176,176a are applied against each other securing not only the patient's limb to the pad, but the cover 150' to the pad, with the cover located between. Thus here, the straps 146,148 have the function both of a closure for the pouch formed by the cover, once it is wrapped around the pad 10, as well as connectors for the pad/cover combination for connecting the patient's limb thereto. At least two connections are needed, in this event. While one, wider strap might appear feasible, since the straps must cross one another behind the pad 10, this cannot be achieved with only one strap and provide any stability. For stability, at least two strap (pairs) are required.

On the other hand, in Figure 7b, an alternative cover 150'a of this type is shown with a single, wide strap 146' having a transverse adhesive strip 176' on which is positioned a pad 10, in the same position shown in Figure 7a. When wrapped around the pad 10, and then around a patient's limb (not shown) on the far side of the cover 150'a, remote from the pad 10, the strip 176' can be adhered, after adjustment for appropriate tightness, to the cover 150'a on its front surface in the region A shown in dotted lines, which generally will be against the rear face of the pad 10, depending on the size of the patient's limb.

A gap 188 remains open at the back of the pad 10, and this may be deemed acceptable. However, it can be minimised simply by widening dimension D (see Figure 7).

Turning to Figure 9, a cover 150" is provided around its periphery with a hem 180 in which a draw-string 182 is threaded. Cover 150" may be provided with holes 184 to allow the motor casing 12 of the vibration pad 10 to protrude through once the cover is fitted. When the draw-string 182 is tightened and tied, the pouch so-formed surrounds the pad 10. However, a gap or hole 186 results at the rear of the vibration pad, and this may, on the one hand, be quite acceptable, since it is really only the front face that is exposed to the possibility of leaks and discharge from an infected limb. On the other hand, such access of biological material may be deemed undesirable, even at the rear face, and consequently, the hole 186 might be minimised, as described above. Alternatively, a preformed pouch, as described above with reference to Figure 6 may be preferred. The drawstring 182, may be elasticated, in which event it may be provided closed and the cover 150" is snap-fitted over the pad 10.

In any event, the cover has straps 146a,b,148a,b as previous embodiments. Here the straps are shown as welded or otherwise connected at 147 to the cover 150". Equally, however, they could be integral, as the straps of the embodiments described above, as indeed, the straps may be welded or otherwise separately connected in the embodiments described above. In Figure 9, the straps are shown arranged so that, when connected together, they would serve to spread the hole 186 and fail to connect the limb securely to the pad. This could be overcome by cross-pieces 188, or by arranging the straps as in the Figures 7a,b and 8 embodiments.

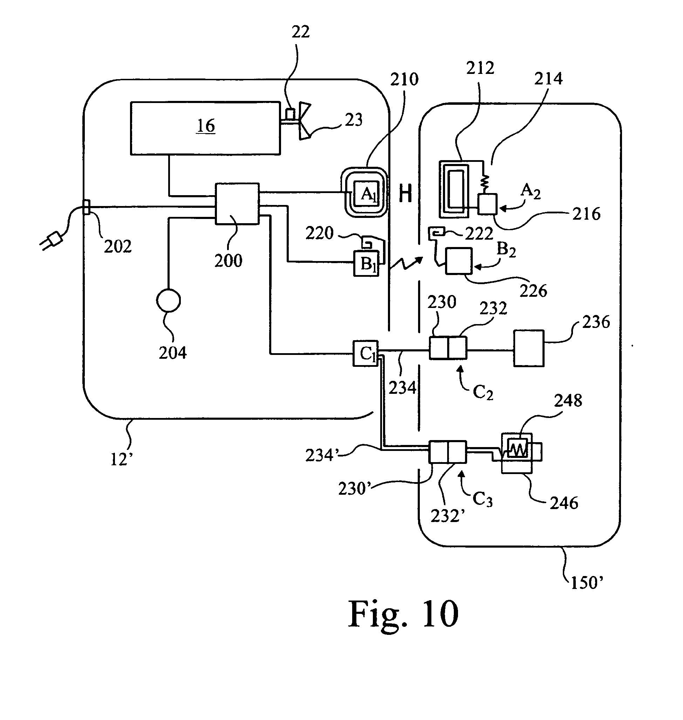

Turning to Figure 10, an alternative arrangement is disclosed in accordance with a second aspect of the present invention. While used properly and appropriately, the arrangement described above is perfectly satisfactory but it does suffer from two potential drawbacks. The first is already mentioned above in that the straps do not allow for any adjustment after a period of time if the straps should be too tight, or too loose. Secondly, the security they provide could simply be overcome, merely by employing some other means to apply pressure between the patient's limb and the vibration pad.

Accordingly, in Figure 10, the vibration device 12' comprises a motor 16 (having its eccentric weight 22 and fan 23 as described above) controlled by a controller 200. The controller is provided with electrical power from a source 202 and supplies that power to the motor 16 when called for by activation of a start signal from device 204. Device 204 may be a button, but may be a device controlled remotely by means not shown.

When device 204 is activated the controller signals one or more of three options or first interfaces A₁, B₁, C₁. In a practical arrangement only one of these options is likely to be employed, although there is equally no reason why they all might not be available in a single device 12'.

A cover 150' is as described above, except here, any integrated straps (not shown) are adjustable by comprising hook and pile fasteners, or peelable and reusable adhesive connections. Consequently, tightness can be adjusted. However, the cover comprises a second interface that corresponds with one of the first interfaces A₁, B₁, C₁. In Figure 10, four are shown, but only one would be employed in a particular cover

Thus, interface A₁,A₂, comprises a magnetic induction link comprising induction loops 210,212 and a disabler circuit 214. The induction loop 212 and circuit 214 are disposed on the cover 150' and the disabler 214 may comprise a smart card type electronic chip 216 that is powered by the induction loop 216 and which modulates the response of the loop 212 so that the information contained in the chip 216 is transmitted to the first induction loop 210 and thence to the controller 200

Interface B₁,B₂ comprises a radio frequency transmission link between antennas 220 and 222, with antenna 222 being incorporated into the cover and being connected to electronic chip 226.

Interface C₁,C₂ comprises a hard wired plug 230 and socket 232 arrangement with an intervening cable connection 234. The socket 232 is provided in the cover 150' and connects to an integrated circuit 236. The plug 230 is one the end of the cable 234 forming part of the vibration device 12'. Of course, which is the plug and which is the socket is not material, nor whether the cable 234 is part of the cover or part of the device 150'.

Finally interface C₁,C₃ also comprises a cable 234', plug 230' and socket 232' arrangement (as interface C₁,C₂,), but here the arrangement further comprises only a simple circuit 246. The arrangements of second interfaces and disablers A₂, B₂ and C₂ are described further below, but interface C₁,C₃ comprises a simple fuse resistor 248. When controller 200 is activated by device 204, it applies a small voltage across the fuse resistor 248 and detects the current though the circuit 246. If the current is within predefined limits, the controller actuates the motor 16. At the same time, the starts a clock (not shown, but which may be part of the controller 200) that counts down a predetermined time. That time may be dependent on the value of the current detected or may be fixed.

At the end of the allotted time, the controller sends a current spike through the circuit 246 that is sufficient to "blow" the fuse 248 and at the same time stops actuation of the motor and enters a restart mode. If the button 204 is activated again, the above procedure repeats except that, on this occasion, no current is detected and consequently the motor 16 does not start. Only if a new cover is applied with an intact fuse 248 will the motor run again. While the fuse is described herein as a resistor, the above principles apply to any component whose response parameters can be altered by a signal from the controller, and so that the controller can detect that those parameters have altered and that accordingly, the cover has been employed for a previous treatment regime.

Returning to interfaces A₁,A₂, B₁,B₂ and C₁,C₂, each, in fact, can employ the same electronic circuit arrangement 216,236,246. In this event, each is an integrated circuit chip of the type employed in smart cards, for example. Not only can such chips provide a unique identification code but also they can store information and therefore be adaptable. For example, they could provide a simple code that enables the controller 200 to actuate the motor, with a counter on the chip noting how long the cover is in use. After a period of time, which may or may not be a single period of time, the code transmitted may change or cease, disabling the controller and stopping it actuating the motor. Alternatively, the clock may be in the controller which, at the end of a treatment period sends a new signal to the chip on the cover which permanently changes the response the chip gives to the first signal. Thus should the pad be disconnected and reconnected to the cover, the pad receives a new code response from the cover which does not enable the controller and it does not activate the motor the start.

Another alternative is that the chip may simply transmit a more complex code when the cover is connected to the pad and the controller is ready to receive the code, and the controller stores the code a memory. Again, after a period of time (counted by either the controller or the chip) the motor might stop and the controller stores the code and does not actuate while a cover having that code is connected to it.

Thus the present invention provides, in several different ways, a system which prevents a cover being used more than once, (or more than a predetermined number of times or for more than a predetermined (cumulative) period of time) whereby the treatment system becomes more practical in that the risks from cross contamination can be reduced.

Throughout the description and claims of this specification, the words "comprise" and "contain" and variations of the words, for example "comprising" and "comprises", means "including but not limited to", and is not intended to (and does not) exclude other moieties, additives, components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith.

## Claims

1. A vibration treatment system comprising a vibration device and a cover (150) therefor, wherein the vibration device comprises a motor (16) driving a vibration element (22) and a pad (110) connected to the motor whereby vibrations caused by rotation of the motor are transmitted to and by the pad (110), and the cover comprises sheet material (152, 154) to protect the pad against contamination when the system is in use and when the cover (150) is applied to the pad, **characterised in that** the system further comprises disablement means to disable use of the vibration device with a particular cover once that cover has been employed in a treatment regime.

2. A vibration treatment system as claimed in claim 1 in which said disablement means comprises a strap integral with the cover suitable for connecting the cover when applied to a pad to the limb of a patient, said strap having a connector that is capable of being secured so as to pressure the pad against the limb and so that, once made, the connection cannot be unmade without disabling the connector against making subsequent connections.

3. A vibration treatment system as claimed in claim 1 or 2 in which said connector comprises a strip of adhesive and said material of the cover is of the type to which the adhesive adheres non-releasably.

4. A vibration treatment system as claimed in claim 3, in which
the adhesive bond, and the cohesive strength of the material adhered, are so strong that the adhesive bond cannot be broken at all, and that, in order to disconnect the pad from a patient's leg, the strap must be broken or cut; or
the adhesive bond is greater than the cohesive strength of the material adhered, whereby, when the strap is disconnected from the cover, a surface layer of the cover or strap is detached, remaining adhered to the adhesive, whereby the strap no longer has capacity to secure the pad to a patient's limb; or
the adhesive bond between the adhesive and the cover material is greater than the cohesive strength of the material of the strap to which the adhesive is adhered, whereby, when the strap is disconnected from the cover, it is the surface layer of the strap that is detached and remains adhered to the adhesive, which itself remains adhered to the cover.

5. A vibration treatment system as claimed in claim 1, in which said disablement means comprises a controller controlling operation of the motor and including a first interface element, and wherein the cover includes a second interface element and a disabler, and wherein said first and second interface elements are interengageable on application of the cover to the pad whereby said controller detects the presence of the cover and enables operation of the motor for a period of time until the controller and disabler disable further operation of the motor until a different cover is applied to the pad.

6. A vibration treatment system as claimed in claim 5, in which:
said first and second interfaces comprise a radio frequency transmission system, and said disabler comprises electric circuitry associated with the cover; or
said first and second, interface comprise induction coils, and said disabler comprises electric circuitry associated with the cover; or
said first and second interfaces comprise a simple plug and socket, and said disabler comprises electric circuitry associated with the cover.

7. A vibration treatment system as claimed in claim 6, in which said electric circuitry comprises an integrated circuit chip, and preferably in which said controller communicates with the chip according to the standard for contactless smart card communications defined in ISO/IEC 14443, 2001.

8. A vibration treatment system as claimed in claim 7, in which said chip includes a unique identification code device that is read by the controller on interengagement of said first and second interfaces and entered into a memory forming part of the controller whereby, if the code is already in the memory the controller disables the motor from operating, whereas if the code is not already in the memory, it is stored in the memory and the motor is enabled to operate for a period of time.

9. A vibration treatment system as claimed in claim 7 or 8, arranged such that, when said first and second interfaces are interengaged the vibration device can transmit a first signal to the chip which on receipt thereof responds with a code that enables the vibration device to commence operation.

10. A vibration treatment system as claimed in claim 9, in which:
a clock in the chip is started on receipt of said first signal and, after a predetermined period of time, said code response is stopped, whence the controller stops the motor in the vibration device; or
a clock in the pad is started on receipt of said code response and, after a predetermined period of time, a second signal is transmitted to the chip so that said code response is stopped, whence the controller stops the motor in the vibration device.

11. A vibration treatment system as claimed in claim 10, in which after stopping said code response a new code is responded to either of said first or second signal, which new code disables the controller from operating the motor.

12. A vibration treatment system as claimed in claim 10 or 11, in which said period of time is a period of time suitable for a single period of therapy using the vibration device.

13. A vibration treatment system as claimed in any preceding claim, in which said covers are tailored for specific uses, having variable treatment time periods, and optionally having different physical characteristics, depending on the use.

14. A vibration treatment system as claimed in claim 13 when dependent on claim 9, in which the code transmitted by the cover informs the vibration device of the treatment regime to be employed, and the vibration device operates at different levels of vibration, or in different modes, depending on the treatment being effected.

15. A vibration treatment system as claimed in claim 6, in which said electric circuitry comprises a fuse resistor, the circuit in the vibration device detecting the resistance of the fuse resistor when connection between the interfaces is made and, provided that the detected resistance is within a predetermined range of resistances, the motor is enabled to operate for predetermined period of time, and preferably in which, at the end of said time period the controller sends a current pulse to the fuse resistor to "blow" the fuse so that it goes into open circuit, thereafter no resistance being detected by the controller and consequently disabling the device.

16. A vibration treatment system as claimed in claim 15, in which the level of resistance of the fuse resistor is employed to distinguish between covers for different applications and to tailor the time of permitted operation of the motor with that cover before the vibration device sends the signal blowing the fuse.

## Patentansprüche

1. Vibrationsbehandlungssystem, das eine Vibrationsvorrichtung und eine Abdeckung (150) dafür umfasst, wobei die Vibrationsvorrichtung einen Motor (16) umfasst, der ein Vibrationselement (22) antreibt, und mit einem Pad (110), das mit dem Motor verbunden ist, wodurch durch die Rotation des Motors verursachte Vibrationen zu dem und durch das Pad (110) übertragen werden, und wobei die Abdeckung ein Lagenmaterial (152, 134) umfasst, um das Pad vor Verunreinigung zu schützen, wenn sich das System im Einsatz befindet und wenn die Abdeckung (150) an dem Pad aufgebracht wird, **dadurch gekennzeichnet, dass** das System ferner eine Deaktivierungseinrichtung umfasst, um den Einsatz der Vibrationsvorrichtung mit einer bestimmten Abdeckung zu deaktivieren, sobald die Abdeckung in einem Behandlungsumfeld eingesetzt wird.

2. Vibrationsbehandlungssystem nach Anspruch 1, wobei die genannte Deaktivierungseinrichtung einen Riemen umfasst, der integral mit der Abdeckung ist sowie geeignet für eine Verbindung der Abdeckung, wenn diese an einem Pad angebracht ist, an einem Körperglied eines Patienten, wobei der genannte Riemen eine Verbindungseinrichtung aufweist, die so gesichert werden kann, dass das Pad gegen das Körperglied gedrückt wird, und so dass, sobald die Verbindung hergestellt worden ist, die Verbindung nicht gelöst werden kann, ohne dass die Verbindungseinrichtung in Bezug auf die Herstellung weiterer Verbindungen deaktiviert ist.

3. Vibrationsbehandlungssystem nach Anspruch 1 oder 2, wobei die Verbindungseinrichtung einen Klebstoffstreifen umfasst, und wobei das genannte Material der Abdeckung so beschaffen ist, dass daran der Klebstoff unlösbar haftet.

4. Vibrationsbehandlungssystem nach Anspruch 3, wobei:
die Klebeverbindung und die Kohäsionsfestigkeit des geklebten Materials so stark sind, dass die Klebeverbindung nicht durchbrochen werden kann, und wobei zur Entfernung des Pads von einem Bein eines Patienten der Riemen zerstört oder zerschnitten werden muss; oder
die Klebeverbindung stärker ist als die Kohäsionsfestigkeit des geklebten Materials, wodurch bei einer Entfernung des Riemens von der Abdeckung eine Oberflächenschicht der Abdeckung oder des Riemens abgelöst wird, die weiter an dem Klebstoff klebt, wodurch der Riemen nicht mehr in der Lage ist, das Pad an einem Körperglied eines Patienten zu sichern; oder
die Klebeverbindung zwischen dem Klebstoff und dem Material der Abdeckung stärker ist als die Kohäsionsfestigkeit des Materials des Riemens, an dem der Klebstoff klebt, wodurch bei einer Entfernung des Riemens von der Abdeckung die Oberflächenschicht des Riemens abgelöst wird und weiter an dem Klebstoff haftet, der selbst weiter an der Abdeckung klebt.

5. Vibrationsbehandlungssystem nach Anspruch 1, wobei die Deaktivierungseinrichtung eine Steuereinrichtung umfasst, welche die Betriebsweise des Motors steuert und ein erstes Schnittstellenelement aufweist, und wobei die Abdeckung ein zweites Schnittstellenelement und einen Deaktivierer aufweist, und wobei die genannten ersten und zweiten Schnittstellenelemente beim Aufbringen der Abdeckung an dem Pad miteinander eingreifen können, wodurch die Steuereinrichtung die Gegenwart der Abdeckung erkennt und den Betrieb des Motors für einen Zeitraum ermöglicht, bis die Steuereinrichtung und der Deaktivierer den weiteren Betrieb des Motors deaktivieren, bis eine andere Abdeckung an dem Pad aufgebracht wird.

6. Vibrationsbehandlungssystem nach Anspruch 5, wobei:
die genannten ersten und zweiten Schnittstellen ein Funkfrequenzübermittlungssystem umfassen, und wobei der genannte Deaktivierer eine der Abdeckung zugeordnete elektrische Schaltkreisanordnung umfasst; oder
die genannten ersten und zweiten Schnittstellen Induktionsspulen umfassen, und wobei der genannte Deaktivierer eine der Abdeckung zugeordnete elektrische Schaltkreisanordnung umfasst; oder
die genannten ersten und zweiten Schnittstellen eine einfache Steckverbindung umfassen, und wobei der genannte Deaktivierer eine der Abdeckung zugeordnete elektrische Schaltkreisanordnung umfasst.

7. Vibrationsbehandlungssystem nach Anspruch 6, wobei die genannte elektrische Schaltkreisanordnung einen integrierten Schaltungschip umfasst, und wobei die genannte Steuereinrichtung vorzugsweise mit dem Chip gemäß der in ISO/IEC 14443, 2001 definierten Norm für kontaktlose Smart Cards kommuniziert.

8. Vibrationsbehandlungssystem nach Anspruch 7, wobei der genannte Chip eine eindeutige Kennzeichnungscodevorrichtung aufweist, die von der Steuereinrichtung beim gegenseitigen Eingriff der genannten ersten und zweiten Schnittstellen gelesen und in einen Speicher eingegeben wird, der einen Teil der Steuereinrichtung bildet, wobei, wenn sich der Code bereits in dem Speicher befindet, die Steuereinrichtung den Betrieb des Motors deaktiviert, wobei, wenn sich der Code noch nicht in dem Speicher befindet, der Code 'n dem Speicher gespeichert wird und der Motor für einen Betrieb über einen Zeitraum aktiviert wird.

9. Vibrationsbehandlungssystem nach Anspruch 7 oder 8, wobei das System so angeordnet ist, dass wenn die genannten ersten und zweiten Schnittstellen miteinander eingreifen, die Vibrationsvorrichtung ein erstes Signal zu dem Chip übermitteln kann, der beim Empfang des Signals mit einem Code antwortet, der es ermöglicht, dass die Vibrationsvorrichtung den Betrieb startet.

10. Vibrationsbehandlungssystem nach Anspruch 9, wobei:
ein Takt in dem Chip beim Empfang des genannten ersten Signals gestartet wird, und wobei nach einem vorbestimmten Zeitraum, die genannte Codeantwort angehalten wird, ab wann die Steuereinrichtung den Motor in der Vibrationsvorrichtung anhält; oder
ein Takt in dem Pad beim Empfang der genannten Codeantwort gestartet wird, und wobei nach einem vorbestimmten Zeitraum, ein zweites Signal zu dem Chip übermittelt wird, so dass die genannte Codeantwort angehalten wird, ab wann die Steuereinrichtung den Motor in der Vibrationsvorrichtung anhält.

11. Vibrationsbehandlungssystem nach Anspruch 10, wobei nach dem Anhalten der genannten Codeantwort ein neuer Code entweder auf das genannte erste oder das genannte zweite Signal als Antwort gegeben wird, wobei der neue Code den Betrieb des Motors durch die Steuereinrichtung deaktiviert.

12. Vibrationsbehandlungssystem nach Anspruch 10 oder 11, wobei es sich bei dem genannten Zeitraum um einen Zeitraum handelt, der sich für einen einzelnen Behandlungszeitraum unter Verwendung der Vibrationsvorrichtung eignet.

13. Vibrationsbehandlungssystem nach einem der vorstehenden Ansprüche, wobei die genannten Abdeckungen für spezifische Verwendungszwecke mit variablen Zeiträumen individuell gestaltet sind, und wobei sie optional unterschiedliche physikalische Eigenschaften aufweisen, abhängig von dem Verwendungszweck.

14. Vibrationsbehandlungssystem nach Anspruch 13, wobei bei einer Abhängigkeit von Anspruch 9, der von der Abdeckung übermittelte Code die Vibrationsvorrichtung über den einzusetzenden Behandlungsbereich informiert, und wobei die Vibrationsvorrichtung mit verschiedenen Vibrationsgraden oder in unterschiedlichen Modi arbeitet, abhängig von der bewirkten Behandlung.

15. Vibrationsbehandlungssystem nach Anspruch 6, wobei die genannte elektrische Schaltkreisanordnung einen Sicherungswiderstand umfasst, wobei die Schaltung in der Vibrationsvorrichtung den Widerstand des Sicherungswiderstands erkennt, wenn eine Verbindung zwischen den Schnittstellen hergestellt wird, und vorausgesetzt dass der erkannte Widerstand innerhalb eines vorbestimmten Widerstandsbereichs liegt, wird der Motor für einen Betrieb für einen vorbestimmten Zeitraum aktiviert, und wobei vorzugsweise am Ende des genannten Zeitraums die Steuereinrichtung einen Stromimpuls an den Sicherungswiderstand sendet, um die Sicherung "auszulösen", so dass diese in eine offene Schaltung wechselt, woraufhin kein Widerstand von der Steuereinrichtung erkannt und die Vorrichtung in der Folge deaktiviert wird.

16. Vibrationsbehandlungssystem nach Anspruch 15, wobei der Wert des Widerstands des Sicherungswiderstands eingesetzt wird, um zwischen Abdeckungen für unterschiedliche Anwendungen zu unterscheiden und zum den Zeitraum für den zulässigen Betrieb des Motors mit dieser Abdeckung individuell festzulegen, bevor die Vibrationsvorrichtung das die Sicherung auslösende Signal sendet.

## Revendications

1. Système de traitement à vibration comprenant un dispositif vibratoire et une enveloppe (150) sur celui-ci, dans lequel le dispositif vibratoire comprend un moteur (16) entraînant un élément vibratoire (22) et un coussin (110) connecté au moteur par lequel les vibrations causées par la rotation du moteur sont transmises à et par le coussin (110), et l'enveloppe comprend une feuille de matériau (152, 134) pour protéger le coussin contre une contamination lorsque le système est utilisé et lorsque l'enveloppe (150) est appliquée sur le coussin, **caractérisé en ce que** le système comprend en outre des moyens de désactivation pour désactiver l'utilisation du dispositif vibratoire avec une enveloppe donnée une fois que l'enveloppe a été utilisée dans un régime de traitement.

2. Système de traitement à vibration selon la revendication 1, dans lequel lesdits moyens de désactivation comprennent une sangle faisant partie intégrante de l'enveloppe appropriée pour connecter l'enveloppe lorsqu'elle est appliquée à un coussin sur le membre d'un patient, ladite sangle ayant un connecteur qui peut être fixé de sorte à faire pression sur le coussin contre le membre et de sorte que, une fois faite, la connexion ne puisse pas être défaite sans empêcher le connecteur de faire des connexions ultérieures.

3. Système de traitement à vibration selon la revendication 1 ou 2, dans lequel ledit connecteur comprend une bande d'adhésif et ledit matériau de l'enveloppe est du type auquel l'adhésif adhère de manière non libérable.

4. Système de traitement à vibration selon la revendication 3, dans lequel
l'adhérence et la force de cohésion du matériau collé sont si fortes que l'adhésif ne peut pas du tout être rompu et que, afin de déconnecter le coussin de la jambe d'un patient, la sangle doit être cassée ou coupée ; ou
l'adhérence est plus grande que la force de cohésion du matériau collé, moyennant quoi, lorsque la sangle est déconnectée de l'enveloppe, une couche de surface de l'enveloppe ou de la sangle est détachée, restant collée à l'adhésif, moyennant quoi la sangle n'a plus la capacité de fixer le coussin sur le membre d'un patient ; ou
l'adhérence entre l'adhésif et le matériau d'enveloppe est supérieure à la force de cohésion du matériau de la sangle à laquelle l'adhésif est collé, moyennant quoi, lorsque la sangle est déconnectée de l'enveloppe, c'est la couche de surface de la sangle qui est détachée et reste collée à l'adhésif, qui lui-même reste collé à l'enveloppe.

5. Système de traitement à vibration selon la revendication 1, dans lequel lesdits moyens de désactivation comprennent un dispositif de commande commandant le fonctionnement du moteur et comprenant un premier élément d'interface, et dans lequel l'enveloppe comprend un second élément d'interface et un dispositif de désactivation, et dans lequel lesdits premier et second éléments d'interface peuvent être imbriqués lors de l'application de l'enveloppe sur le coussin, moyennant quoi ledit dispositif de commande détecte la présence de l'enveloppe et permet le fonctionnement du moteur pendant une période de temps jusqu'à ce que le dispositif de commande et le dispositif de désactivation désactivent le fonctionnement du moteur jusqu'à ce qu'une enveloppe différente soit appliquée sur le coussin.

6. Système de traitement à vibration selon la revendication 5, dans lequel :
lesdites première et seconde interfaces comprennent un système de transmission radio fréquence et ledit dispositif de désactivation comprend des circuits électriques associés à enveloppe ; ou
lesdites première et seconde interfaces comprennent des bobines d'induction et ledit dispositif de désactivation comprend des circuits électriques associés à l'enveloppe ; ou
lesdites première et seconde interfaces comprennent une fiche mâle et femelle simple et ledit dispositif de désactivation comprend des circuits électriques associés à l'enveloppe.

7. Système de traitement à vibration selon la revendication 6, dans lequel lesdits circuits électriques comprennent une puce de circuit intégré, et de préférence dans lequel ledit dispositif de commande communique avec la puce selon la norme pour les communications par carte à puce sans contact définie dans ISO/CEI 14443, 2001.

8. Système de traitement à vibration selon la revendication 7, dans lequel ladite puce comprend un dispositif de code d'identification unique qui est lu par le dispositif de commande lors de l'imbrication desdites première et seconde interfaces et est entré dans une mémoire faisant partie du dispositif de commande moyennant quoi si le code est déjà dans la mémoire, le dispositif de commande désactive le fonctionnement du moteur, alors que si le code n'est pas déjà dans la mémoire, il est stocké dans la mémoire et le moteur est activé pour fonctionner pendant une période de temps.

9. Système de traitement à vibration selon la revendication 7 ou 8, agencé de telle sorte que, lorsque lesdites première et seconde interfaces sont imbriquées, ledit dispositif vibratoire peut transmettre un premier signal à la puce qui à sa réception répond avec un code qui permet au dispositif vibratoire de commencer à fonctionner.

10. Système de traitement à vibration selon la revendication 9, dans lequel :
une horloge dans la puce est démarrée à la réception dudit premier signal et, après une période de temps prédéterminée, ladite réponse de code est arrêtée, en conséquence de quoi le dispositif de commande arrête le moteur dans le dispositif vibratoire ; ou
une horloge dans la puce est démarrée à la réception de ladite réponse de code et, après une période de temps prédéterminée, un second signal est transmis à la puce de sorte que la réponse de code est arrêtée, en conséquence de quoi le dispositif de commande arrête le moteur dans le dispositif vibratoire.

11. Système de traitement à vibration selon la revendication 10, dans lequel, après l'arrêt de ladite réponse de code, il est répondu à un nouveau code par ledit premier ou second signal, lequel nouveau code désactive le dispositif de commande qui arrête le fonctionnement du moteur.

12. Système de traitement à vibration selon la revendication 10 ou 11, dans lequel ladite période de temps est une période de temps appropriée pour une seule période de thérapie en utilisant le dispositif vibratoire.

13. Système de traitement à vibration selon l'une quelconque des revendications précédentes, dans lequel lesdites enveloppes sont adaptées pour des utilisations spécifiques, ayant des périodes de temps de traitement variables, et en option ayant différentes caractéristiques physiques, selon l'usage.

14. Système de traitement à vibration selon la revendication 13 telle que dépendant de la revendication 9, dans lequel le code transmis par l'enveloppe informe le dispositif vibratoire du régime de traitement à utiliser, et le dispositif vibratoire fonctionne à différents niveaux de vibration, ou dans différents modes, en fonction du traitement effectué.

15. Système de traitement à vibration selon la revendication 6, dans lequel lesdits circuits électriques comprennent une résistance de protection, le circuit dans le dispositif vibratoire détectant la résistance de la résistance de protection lorsque la connexion entre les interfaces est faite et, à condition que la résistance détectée soit dans une plage de résistances prédéterminée, le moteur est activé pour fonctionner pendant une période de temps prédéterminée, et de préférence dans lequel, à la fin de ladite période de temps le dispositif de commande envoie une impulsion de courant à la résistance de protection pour « faire sauter » le fusible de sorte qu'il passe en circuit ouvert, par la suite aucune résistance n'étant détectée par le dispositif de commande et par conséquent le dispositif étant désactivé.

16. Système de traitement à vibration selon la revendication 15, dans lequel le niveau de résistance de la résistance de protection est utilisé pour faire la distinction entre les enveloppes pour différentes applications et pour adapter le temps de fonctionnement autorisé du moteur avec cette enveloppe avant que le dispositif vibratoire n'envoie le signal faisant sauter le fusible.
